# EUROPEAN PATENT SPECIFICATION

(11) **EP 4 287 833 B1**
(45) Date of publication and mention of the grant of the patent: **04.11.2026**
(21) Application number: 22703849.4
(22) Date of filing: 02.02.2022
(51) Int. Cl.: A21D 8/04, A23L 29/212, A23L 33/21, C12N 9/10, C12P 19/18, A21D 13/064, A21D 13/066, A23L 29/00

(54) **NOVEL ANTI-STALING ENZYME, AND METHODS, DOUGHS AND BAKED FOOD PRODUCTS RELATING THERETO**
NEUES ENZYM ZUR VERHINDERUNG VON ALTBACKENWERDEN UND VERFAHREN, TEIGE UND BACKWARENPRODUKTE IM ZUSAMMENHANG DAMIT
NOUVELLE ENZYME ANTI-RASSISSEMENT ET PROCÉDÉS, PÂTES ET PRODUITS ALIMENTAIRES CUITS ASSOCIÉS

(30) Priority: 02.02.2021 EP 21154734
(43) Date of publication of application: 13.12.2023
(73) Proprietor: Coöperatie Koninklijke Avebe U.A., 9641 GK Veendam (NL)
(72) Inventor: DUISTERWINKEL, Wouter Jan, 9641 GK Veendam (NL); LEEMHUIS, Reinder Johannes, 9641 GK Veendam (NL)
(74) Representative: V.O.
(86) International application number: PCT/NL2022/050048
(87) International publication number: WO 2022/169357

(56) References cited:
- EP-A1- 2 248 907
- WO-A1-2017/062687
- US-A1- 2019 119 712
- DATABASE Geneseq [online] 1 June 2017 (2017-06-01), "Bacillus coagulans 2-6 alpha-glucanotransferase protein SEQ ID NO: 7.", XP002803592, retrieved from EBI accession no. GSP:BDV43072 Database accession no. BDV43072
- LI DAN ET AL: "Effects of Streptococcus thermophilus GtfB enzyme on dough rheology, bread quality and starch digestibility", FOOD HYDROCOLLOIDS, ELSEVIER BV, NL, vol. 96, 6 May 2019 (2019-05-06), pages 134 - 139, XP085738269, ISSN: 0268-005X, [retrieved on 20190506], DOI: 10.1016/J.FOODHYD.2019.05.009
- REIS RODRIGO A ET AL: "A new pullulan and a branched (1C3)-, (1C6)-linked L-glucan from the lichenised ascomycete Teloschistes flavicans", FEMS MICROBIOLOGY LETTERS, vol. 210, 1 January 2002 (2002-01-01), pages 1 - 5, XP055935728
- TE POELE EVELIEN M. ET AL: "GtfC Enzyme of Geobacillus sp. 12AMOR1 Represents a Novel Thermostable Type of GH70 4,6-[alpha]-Glucanotransferase That Synthesizes a Linear Alternating ([alpha]1 -> 6)/([alpha]1 -> 4) [alpha]-Glucan and Delays Bread Staling", JOURNAL OF AGRICULTURAL AND FOOD CHEMISTRY, vol. 69, no. 34, 24 August 2021 (2021-08-24), US, pages 9859 - 9868, XP055935679, ISSN: 0021-8561, DOI: 10.1021/acs.jafc.1c03475
- YANG WEIKANG ET AL: "Directed Mutation of Two Key Amino Acid Residues Alters the Product Structure of the New 4,6-[alpha]-Glucanotransferase from Bacillus sporothermodurans", JOURNAL OF AGRICULTURAL AND FOOD CHEMISTRY, vol. 69, no. 48, 30 November 2021 (2021-11-30), US, pages 14680 - 14688, XP055935736, ISSN: 0021-8561, DOI: 10.1021/acs.jafc.1c05263

## Description

The invention relates to the field of food technology and anti-staling enzymes.

Bread staling is a complex process involving numerous chemical and physical changes in the bread which have deleterious effects on the taste, texture, aroma, crumb structure, and mouthfeel of the product. While several models have been proposed to explain staling, it may be generally said to arise as a result of starch retrogradation, water loss from the bread, and water migration within the bread.

The staling of bread is an important issue in the baking industry because it imposes sharp limitations on the shelf life of bread products. To mitigate the impact of staling, special storage and packaging of bread is typically employed, which is costly and can be unattractive to the consumer. Such measures provide moderate improvement in shelf life, but even under optimal storage conditions, such as sealing the bread in a high humidity environment, most bread will begin to stale after only a few days. Improved packaging cannot eliminate staling.

Further, special packaging is not possible for the segment of the industry that desires to offer unpackaged products, including, for example, smaller bakeries which market "fresh baked" products, donut shops which offer their products on racks or the like, as well as retail grocery stores which are increasingly exploiting the market for unpackaged baked goods, such as bread loafs, muffins, bagels, donuts, and cookies. Often, the shelf life of unpackaged bread products is measured in hours and the entire inventory must be discarded and replaced with fresh product one or more times throughout the day. Therefore, preventing or slowing the staling of bread products provides a significant economic advantage.

The mechanism underlying the staling of bread is not completely understood, and many factors are considered to contribute to this phenomenon. One of these factors is the change in the structure of the starch inside the bread. During the kneading process, gluten forms and starch absorbs moisture. As the bread bakes, the starch gelatinizes. Immediately after baking, the starch is still gelatinous, which gives the bread a nice soft, elastic crumb. Over time, the starch recrystallizes. As it crystallizes, it also traps the water in the bread. These two things make the crumb firm and inelastic. This leads to a hard and dry bread.

To delay staling, to improve texture of bakery products, several additives may be used in bread baking. These include chemicals, sugars, enzymes or combinations of these. Well-known additives are: milk powder, gluten, emulsifiers (mono- or diglycerides, sugar esters, lecithin, etc.), granulated fat, oxidants (ascorbic acid or potassium bromate), cysteine, sugars or salts. Advances in biotechnology have made 'new' enzymes available for the industry. Since enzymes are produced from natural ingredients, they will find greater acceptance by the consumers because of their demand for products without chemicals. Several enzymes have been suggested to act as dough and/or bread improvers, by modifying one of the major dough components. Enzymes active on starch have been suggested to act as anti-staling agents. Examples are: alpha-amylases, branching and debranching enzymes, maltogenic amylases, beta-amylases and amyloglucosidases.

Commonly used enzymes include xylanases and maltogenic amylases. A maltogenic amylase can slow the recrystallization process and extend the time that the bread is soft and elastic. It does this by continuously breaking down the starch chains. Thermostable maltogenic amylases hydrolyse the external carbohydrate chains of starch molecules thereby releasing maltose. Maltose is a reducing sugar which may have an effect on the organoleptic and glycemic properties of the baked product. In (gluten) free bread the release of maltose may induce a sweeter taste and due to its easy digestibility it may increase the glycemic index of the baked products.

Xylanases are hydrolytic enzymes, which randomly cleave the beta-1,4 backbone of arabinoxylans from plant cell walls. Different species of Aspergillus and Trichoderma produce these enzymes. Xylanases have been found to improve the bread volume, crumb structure and reduce stickiness rather than that they tend to have an anti-staling effect.

The present inventors aimed at identifying novel anti-staling enzymes. In particular, they sought to provide anti-staling enzymes which improve the properties of the baked dough products and provide a better baked product without releasing reducing sugars. To allow for sufficient enzyme activity during the bread preparation, the enzyme must exhibit some degree of thermostability.

Surprisingly, these goals were met by the biochemical characterization of a putative GtfC-like enzyme of the *Geobacillus* sp. 12AMOR1. Pure and active GtfC AMOR enzymes (8 constructs) were obtained by heterologous expression in *E. coli.* Biochemical characterization of the smallest C-truncated variant, GtfC AMOR738 (726 AA; 82 kDa) showed that it has an optimum temperature for activity of 60°C. This thermostable 4,6-α-glucanotransferase has a novel product specificity, cleaving off predominantly maltose units from amylose, attaching them with an α(1 → 6)-linkage to acceptor substrates. Detailed structural characterization of its starch-derived α-glucan products revealed that it yields a unique polymer with alternating α(1 → 6)/α(1 → 4)-linked glucose units but without branches. In fact, this enzyme represents a novel maltogenic α-amylase in the sense that it also cleaves off maltose units. The incorporation of this GtfC-type 4,6-α-glucanotransferase enzyme in a farinaceous dough (either wheat-containing or gluten-free) was found to confer desirable properties to the resulting bread. In particular, enzyme addition significantly reduced the crumb hardness in a dose-dependent fashion.

Accordingly, the invention relates to a method of preparing a baked food product by baking a farinaceous dough, comprising incorporating into the dough a thermostable GtfC-type 4,6-α-glucanotransferase enzyme, capable of transferring a maltose moiety from a polysaccharide or oligosaccharide substrate to the non-reducing end of an oligosaccharide acceptor via a new α(1 →6) linkage without forming α(1→ 4,6) branching points.

The invention also relates to a farinaceous dough comprising a thermostable GtfC-type 4,6-α-glucanotransferase enzyme capable of transferring a maltose moiety from a polysaccharide or oligosaccharide substrate to the non-reducing end of an oligosaccharide acceptor via a new α(1→6) linkage without forming α(1→4,6) branching points. In another aspect the invention relates to preparing a baked product comprising a carbohydrate comprising alternating α(1→6)/α(1→4) glycosidic bonds.

The word "farinaceous" is derived from the Latin word farina, which means flour. Wheat flour is largely made up of starch. Accordingly, the term "farinaceous dough" refers to any dough that contains starch and therewith a polysaccharide or oligosaccharide substrate for the GtfC-type 4,6-α-glucanotransferase enzyme.

In one embodiment, the invention provides a method of preparing a baked food product by baking a farinaceous dough, comprising incorporating into the dough a GtfC-type 4,6-α-glucanotransferase enzyme, wherein the GtfC-type enzyme (i) is capable of transferring a maltose moiety from a polysaccharide or oligosaccharide substrate to the non-reducing end of an oligosaccharide acceptor via a new α(1→6) linkage without forming α(1→ 4,6) branching points, and (ii) has an activity optimum in the temperature range of 50-70°C determined at pH 6with amylose V as substrate.

Also provided is farinaceous dough comprising a GtfC-type 4,6-α-glucanotransferase enzyme, wherein the GtfC-type enzyme (i) is capable of transferring a maltose moiety from a polysaccharide or oligosaccharide substrate to the non-reducing end of an oligosaccharide acceptor via a new α(1→6) linkage without forming α(1→ 4,6) branching points, and (ii) has an activity optimum in the temperature range of 50-70°C determined at pH 6 with amylose V as substrate.

In one aspect, the thermostable GtfC-type enzyme has an optimum pH in the range of 4.0 to 7.5. This low pH is particularly relevant for application in or with sourdough. Sourdough, a traditional fermented dough, is made via natural fermentation by lactic acid bacteria (LAB). Its pH changes from near neutral to acid during the subculture process. Preferably, the pH optimum of the GtfC-type enzyme is in the range of 5.0 to 7.0. In a specific embodiment, the pH optimum of the GtfC-type enzyme is 5.5-6.5.

Preferably, the thermostable GtfC-type enzyme for use in the present invention has an optimum temperature for enzyme activity in the range of 55-65°C. Thermostability may also be expressed as the amount of activity which remains after the enzyme has been exposed to elevated temperature, e.g. in the range of 50-70°C. After cooling, the remaining activity is then suitably determined by measuring the initial rate of substrate conversion (e.g. Amylose V) at about 40-45°C. For example, enzyme (40-50 µg/mL) is incubated in a reaction buffer in the absence of amylose V for 0, 10, 30 and 60 min at 60 °C and then immediately cooled to 4 °C. The residual enzyme activity (initial rate) of the heat-treated enzyme preparations is then measured at 40 °C using 0.125% (w/v) Amylose V. In one embodiment of the invention, the GtfC-type enzyme has at least 80%, preferably at least 85%, more preferably at least 90%, residual 4,6-α-glucanotransferase activity after incubating the enzyme in a 25 mM sodium citrate buffer containing 1 mM CaCl₂ at a temperature of 60°C for 60 min.

A method or dough comprising a thermostable GtfC-type 4,6-α-glucanotransferase enzyme as herein disclosed in not known or suggested in the art.

As is demonstrated herein below, a GtfC-type enzyme for use in the present invention has as main catalytic activity the transfer of a maltose residue to the non-reducing end of an oligosaccharide. This modification of non-reducing ends is advantageous for suppressing retrogradation. As a side-reaction, some hydrolysis may occur resulting in the formation of small amounts of free maltose. This side reaction also takes place when using GtfB-type enzymes. However, because these are glucose-transferring enzymes, the side product will be glucose. For example, *Lactobacillus reuteri* 121 GTFB enzyme synthesizes linear glucan chains composed of α(1→6) linked glucoses attached at the nonreducing end of α(1→4) oligosaccharides (Kralj et al., Appl Environ Microbiol. 2011;77(22):8154-63.4). Since the perceived sweetness of maltose is about two times less than that of sucrose, the use of a GtfC-type maltose-transferring enzyme of the present invention provides for the modification of non-reducing ends, while minimizing the development of an undesired sweet taste of the baked product.

The maltose-transferring activity can be readily determined by methods known in the art. For example, it can be determined as described in example 3, by quantifying the conversion of amylose V in time using iodine staining. Since this assay only reveals that amylose is consumed (also the catalytic activity of alpha-amylase, branching enzyme, maltogenic amylase, et cetera can be measured with this assay) the structural properties of the carbohydrate products formed have to be elucidate to know the reaction specificity of the enzyme. These methods are described in example 5. In case of the GtfC the maltose transferase activity follows from the observations that its products are digested by pullulanase forming maltose, but not by alpha-amylase and dextranase. And secondly, the methylation analysis demonstrates that the product is composed of α(1→4) and α(1→6) linked glucoses, and does not contain α(1→4,6) branches. Once the reaction specificity is known, the iodide staining assay is a fast and convenient method to quantify the catalytic activity of the enzyme.

EP228907A1 discloses the use of a 4,6-alpha-glucanotransferase of the GtfB type to prepare gluco-oligosaccharides that are used in food products such as baked products. The enzyme has an optimal activity at pH 4.7 and 37°C. The structures of the glycans synthesized are characterized by one or more consecutive α(1→6) glucosidic linkages, and one or more α(1→4) glucosidic linkages, wherein the term "consecutive" means that there are at least two repeated linkages of the same type, i.e. two or more α(1→6) glucosidic or two or more α(1→4) glucosidic linkages to follow one after another, without an interruption. See exemplary glycans illustrated in Figure 1 of EP228907A1. This is in marked contrast to the glucans according to the present invention, having alternating α(1→6)/α(1→4) glycosidic bonds and having less than 0.5% consecutive α(1→6) glycosidic bonds and less than 0.5% consecutive α(1→4) glycosidic bonds.

US2019/119712A1 relates to a GtfB-type enzyme in the preparation of a wheat flour dough and bread. It fails to disclose a GtfC-type 4,6-α-glucanotransferase enzyme that has an optimum temperature in the range of 50-70°C determined at pH 6 with amylose V as substrate. Rather, US2019/119712A1 teaches that the assay conditions for its enzyme are 40°C and pH 6. Like EP228907A1, in case of US2019/119712A1 the GtfB-type enzyme shows a significant lower temperature optimum than what is sought for in thermostable enzymes.

WO2017/062687 discloses a new GH70 subfamily of alpha-glucanotransferases (α-GT) which is distinct from 4,6- α-glucanotransferase enzymes. α-GT is capable of synthesizing a glucooligo mixture including isomalto-oligosaccharides, which have beneficial health effects as prebiotic. WO2017/062687 is silent about adding α-GT to a dough or its application as anti-staling enzyme.

US 2019/330671 generally relates to method of producing an α-glucan containing α(1→3) linked D-glucose units using α-glucanotransferase of the GTFB, GTFC and GTFD type, and to the use of the α-glucanotransferase enzyme for reducing the digestible carbohydrates of a starch containing food material. According to US 2019/330671, the substrate, e.g. selected from the group consisting of starch, starch derivatives, malto-oligosaccharides, gluco-oligosaccharides, amylose, amylopectin, maltodextrins, α(1→4) glucans, may be comprised within a cereal flour. Notably however, US 2019/330671 is silent about farinaceous doughs and method of preparing a baked food product thereof and only exemplifies one GtfB-type enzyme. Unlike the enzyme of the present invention, the GtfB-type enzymes have a glucose-transferase activity. Furthermore, the alpha-glucans produced are characterized among others by having α(1→4, 6) branching points.

The only GtfC-type enzyme referred to in US 2019/330671 is GtfC from *E. sibiricum* 255-15, which is not thermostable and lacks the capacity to transfer a maltose moiety from a polysaccharide or oligosaccharide substrate to the non-reducing end of an oligosaccharide acceptor via a new α(1→6) linkage without forming α(1→4,6) branching points. See Gangoiti et al. (Appl Environ Microbiol. 2016;82(2):756-66), disclosing that this enzyme has an optimum activity at 45°C, which activity decreases drastically when the reaction is carried out at 55°C. Hence, even if US 2019/330671 were considered to implicitly disclose the incorporation of enzyme into a farinaceous dough and prepare a bread, it fails to provide suggest using a thermostable GtfC-type enzyme having maltose-transferase activity for that purpose.

Li et al. (Food Hydrocoll. Elsevier BV, NL, vol. 96, 6 May 2019, pages 134-1395) studied the effects of *Streptococcus thermophilus* GtfB (StGTFB) enzyme on dough rheology, bread quality and starch digestibility. Although StGTFB also forms α(1→6) linkages, it does not create linear chains by elongating at the nonreducing end, but it generates branches α(1→4,6) with chains (5). Thus, StGTFB behaves as starch/glycogen branching enzyme and does not modify non-reducing ends. The GtfC-type enzyme for use in the present invention is different from the GTFB-type enzymes, attaching maltose units to the nonreducing end of α(1→4) oligosaccharides via α(1→6) linkages.

The skilled person will be able to determine the dosage of the GtfC-type enzyme in a farinaceous dough. In one embodiment, it is incorporated in the dough in an amount of 50-100,000, preferably 100 - 50,000, more preferably 500 - 20,000 U per kg of the total weight of starch in the dough. Exemplary dosages include at least 600, 800, 1000, 2000, 4000, 6000, 8000, 9000 or 10,000 U per kg of the total weight of starch. As used herein, the activity of the enzyme in Units is defined as the amount of amylose (mg) that is converted by one mg of enzyme per minute. For example, good anti-staling results (e.g. reduced hardness) can be obtained when the enzyme is incorporated in a farinaceous dough in the range of 100 to 15,000 U per kg starch, preferably 500 to 15,000, or 1000 to 13,000, or 4000 to 12,000 U/kg starch.

The GtfC-type enzyme can be a polypeptide that is encoded by a DNA sequence that is found in a non-lactic acid Gram-positive bacterium, preferably a bacterium selected from the genera *Bacillus* and *Geobacillus.* For example, it is a polypeptide that is encoded by a DNA sequence that is found in *Geobacillus* sp. 12AMOR1, or a functional homolog or fragment thereof having the defined GtfC-type 4,6-α-glucanotransferase activity.

In one embodiment, theGtfC-type enzyme is a polypeptide having accession number AKM18207, or a functional homolog or fragment thereof having GtfC-type 4,6-α-glucanotransferase activity.

Very good results can be obtained using GFTC-type 4,6-α-glucanotransferase enzyme having accession number AKM18207, or a functional homolog or fragment thereof having the specified enzymatic activity. This enzyme is found in *Geobacillus* sp. 12AMOR1 (deposit number DSM 17290 at the DSMZ),

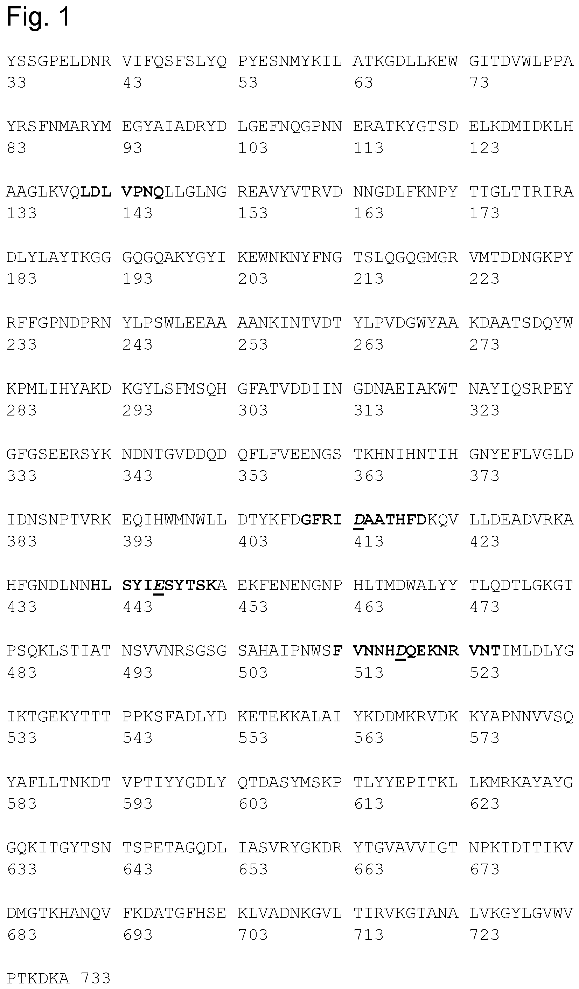

Preferably, the enzyme comprises an amino acid sequence having at least 80% sequence identity to Sequence 1 (see Figure 1), representing residues 33-738 of the GH70 catalytic core of the GtfC-type 4,6-α-glucanotransferase of the *Geobacillus* sp. 12AMOR1 (GenBank accession number AKM18207), and showing the desirable catalytic activity for use in the present invention. This sequence does not contain the N-terminal residues 1-32 representing the signal sequence of the full length enzyme.

For example, the enzyme shows at least 85, 90, 95, 96, 97, 98, or 99% identity to Sequence 1. In one embodiment, the 4,6-α-glucanotransferase may consist of an amino acid sequence having at least 80% identity to Sequence 1, for example at least 85, 90, 95, 96, 97, 98, or 99% identity to Sequence 1.

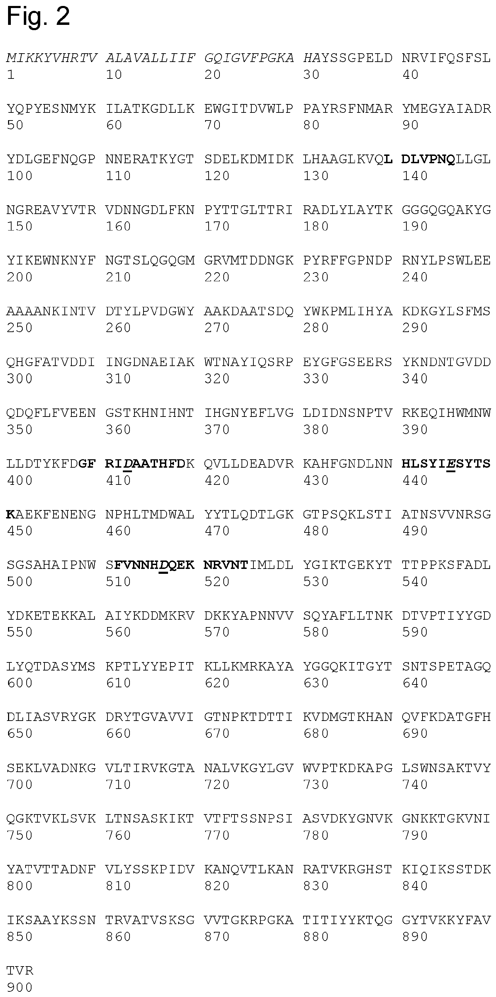

Whereas for recombinant production of enzymes it is often preferred to minimize the size of the expressed polypeptide, the catalytic GH70 core sequence may be extended at the N- and/or C-terminus if so desired. For example, up to 165 C-terminal amino acid residues as found in the full length wild-type enzyme (see Sequence 2; Figure 2) can be added.

Accordingly, in one embodiment the invention provides a method of preparing a baked food product by baking a farinaceous dough, comprising incorporating into the dough a GtfC-type 4,6-α-glucanotransferase enzyme, wherein the GtfC-type enzyme shows at least 85, 90, 95, 96, 97, 98, or 99% identity to Sequence 1 or 2.

In one embodiment, the GtfC-type enzyme is a polypeptide comprising the conserved motifs I, II, III and IV of the GTFC-like members of the GH70 family, wherein the motifs have the following amino acid sequences:
Motif I: [M/E/L]DLVPNQ, preferably LDLVPNQ
Motif II: GFRIDAA[S/G/T]H[Y/F]D, preferably GFRIDAATHFD
Motif III: HLSYIESY[K/S/Q/T][S/N][E/V/A/K], preferably HLSYIESYTSK
Motif IV: FV[N/T]NHDQEKNR[V/I]N[Q/N/T], preferably FVNNHDQEKNRVNT.

For example, the GtfC-type enzyme comprises one or more of the sequences LDLVPNQ, GFRIDAATHFD, HLSYIESYTSK and FVNNHDQEKNRVNT.

In a preferred aspect, the GtfC-type 4,6-α-glucanotransferase enzyme comprises residues 413Asp, 446Glu and 519Asp that constitute the catalytic triad of the GH70 enzyme family. See Figure 2 of Gangoiti *et al.* See also the sequences of Figures 1 and 2, showing the conserved motifs in bold and the 3 catalytic residues in italics and underlined font.

Useful C-terminally truncated enzyme variants for use in the present invention include those having or consisting of amino acids 33-902 (example 1), 33-738 (Sequence 1), 33-739, 33-748, 33-752, 33-753 or 33-770 of the GtfC-type 4,6-α-glucanotransferase having accession number AKM18207 of the *Geobacillus* sp. 12AMOR1. Accordingly, the 4,6-α-glucanotransferase enzyme may comprise an amino acid sequence having at least 80%, preferably at least 85%, sequence identity to Sequence 2. Also, the full length enzyme, optionally without the N-terminal signal peptide sequence, can be used.

As will be understood by a person skilled in the art, the GtfC-type enzyme for use in the present invention may contain one or more protein tags to aid in the expression, isolation and/or purification after recombinant production. In one embodiment, the enzyme comprises an N-terminal His-tag sequence, for example MAHHHHHHSAALEVLFQGPG.

A method of the invention for preparing a baked bread comprises preparing a farinaceous dough which comprises a GtfC-type 4,6-α-glucanotransferase enzyme as defined in claim 1. Typically, the dough composition is prepared by combining flour, water, yeast and/or other leavening agents, GtfC-type 4,6-α-glucanotransferase enzyme and optionally other bakery ingredients. For example, edible salt, sugar, oils, fats, emulsifiers, flavorings and the like can be added.

In one embodiment, the dough is a wheat flour dough composition. For example, the invention provides a dough comprising wheat flour, GtfC-type 4,6-α-glucanotransferase enzyme, yeast, sugar, sodium chloride and a vegetable oil, such as sunflower oil. The type of wheat flour is generally free from any additives. It may however contain some extra alpha-amylase to match the average natural concentration according to the manufacturer. In a specific embodiment, the invention provides a wheat dough composition comprising wheat flour, GtfC-type 4,6-α-glucanotransferase enzyme, instant yeast, sugar, sodium chloride and sunflower oil.

In another embodiment, the dough is a gluten-free or gluten-reduced dough comprising one or more gluten-free of gluten-reduced flour(s) and/or starches, GtfC-type 4,6-α-glucanotransferase enzyme, yeast, a protein such as potato protein, a starch, such as potato starch, a hydrocolloid, such as sodium carboxymethylcellulose (CMC), a fiber, such as potato fiber, sugar, sodium chloride and a vegetable oil, such as sunflower oil. Examples of suitable gluten-free (cereal) flour include rice flour, buckwheat flour, corn flour, millet flour, amaranth flour, teff flour, oat flour, quinoa flour, sorghum flour, soy flour, pea flour, chia flour, chickpea flour, lentil flour, tapioca flour and potato flour, either singly or as a mixture.

Examples of suitable gluten-free (cereal) starch include (pregelatinized) (waxy) potato starch, (pregelatinized) wheat starch (gluten-free), (pregelatinized) (waxy) corn starch, (pregelatinized) (waxy) rice starch and (pregelatinized) tapioca starch, either singly or as a mixture. Examples of suitable proteins include potato protein, soy protein, chickpea protein, lupin protein, egg protein, whey protein, canola protein, lentil protein, faba bean protein, pea protein, either singly or as a mixture. Examples of suitable hydrocolloids include sodium carboxymethylcellulose (CMC), hydroxypropyl methylcellulose (HPMC), xanthan gum, guar gum, locust bean gum, alginate, and carrageenan, either singly or as a mixture. Examples of suitable fibers include potato fiber, inulin, psyllium husk, beta-glucan, citrus fiber, apple fiber, bamboo fiber and dextran, either singly or as a mixture.

In a specific embodiment, the invention provides a gluten-free dough composition comprising GtfC-type 4,6-α-glucanotransferase enzyme as defined in claim 1, a gluten-free or gluten-reduced flour, such as rice flour, and at least one potato starch, preferably selected from native potato starch, fine granulated potato starch and extruded instant potato starch, or any combination thereof. The dough may furthermore comprise insoluble potato fiber and/or potato protein.

In one embodiment the product is a high moisture bakery product such as a (gluten-free) cake, muffin or a donut. Comprising (gluten-free) flour, (gluten-free) starches, sugar, eggs and/or other proteins, oil/fat such as butter, hydrocolloids, emulsifiers, leavening agents, flavorings, milk (products) such as whey protein, sodium chloride and water. In a specific embodiment the invention provides a cake batter composition comprising a GtfC-type 4,6-α-glucanotransferase enzyme as defined in claim 1, heat treated wheat flour, sugar, butter, eggs, potato starch, mono- and diglycerides of fatty acids, polyglycerol esters of fatty acids, sodium bicarbonate, sodium acid pyrophosphate (SAPP), whey protein, xanthan and vanilla aroma.

A farinaceous dough of the invention can be processed into a baked product using conventional bread baking methods. Typically, the dough is fermented/leavened, e.g. at temperatures between 20-35°C, prior to baking at a temperature in excess of 180°C.

The invention also provides a baked bread that is obtainable or obtained by a method according to the invention, or by baking a dough as herein disclosed. A bread of the invention is among others characterized in that it has an improved shelf-life, crumb structure, lower crumb hardness and longer lasting softness. In one aspect, the bread shows delayed staling during (cold) storage. In one embodiment, the bread is a wheat bread. In a preferred embodiment, the bread is a gluten-free bread.

A further aspect of the invention relates to a method to increase the shelf-life of a bread product, the method comprising incorporating into a farinaceous bread dough a GtfC-type 4,6-α-glucanotransferase enzyme as defined in claim 1, and baking the dough to obtain a bread product. As used herein, the shelf-life refers to the sensorial/textural properties of the bread product rather than the microbiological status.

Still further, the invention provides the use of a GtfC-type 4,6-α-glucanotransferase enzyme as defined in claim 1 as a bread-improver or anti-staling agent in a baked dough product. The use comprises incorporating the GtfC-type 4,6-α-glucanotransferase enzyme as defined herein above into the dough prior to baking.

In one embodiment, it provides the use of a GtfC-type 4,6-α-glucanotransferase enzyme as defined in claim 1 to improve or preserve at least one property of a baked dough product, preferably to improve or preserve one or more of loaf volume, crumb structure, crumb resilience, crumb springiness, dough strength, dough stability and crumb softness.

In a further aspect, the invention discloses a method for providing a linear alpha-glucan having alternating α(1→4) and α(1→6) glucosidic linkages, comprising contacting a polysaccharide or oligosaccharide substrate comprising at its non-reducing end at least two α(1→4) linked D-glucose units with a GtfC-type 4,6-α-glucanotransferase as herein disclosed, which is capable of transferring a maltose moiety from the polysaccharide or oligosaccharide substrate to the non-reducing end of an oligosaccharide acceptor via a new α(1→6) linkage without forming α(1→4,6) branching points. Preferably, the method uses the GtfC-type enzyme from *Geobacillus* sp. 12AMOR1 (deposit number DSM 17290 at the DSMZ), having GenBank accession number AKM18207, or a functional homolog or fragment thereof having the specified enzymatic activity.

In one embodiment, the substrate has a degree of polymerization of at least three (DP ≥3), preferably at least four DP ≥4). The substrate is suitably selected from the group consisting of starch, waxy starch, high amylose starch, starch derivatives, malto-oligosaccharides, amylose, amylopectin, maltodextrins, α(1→4) glucans, reuteran, or combinations thereof.

The method may comprise contacting the polysaccharide or oligosaccharide substrate with the 4,6-a-glucanotransferase enzyme at a temperature of at least 45°C, preferably between 50°C and 75°C.

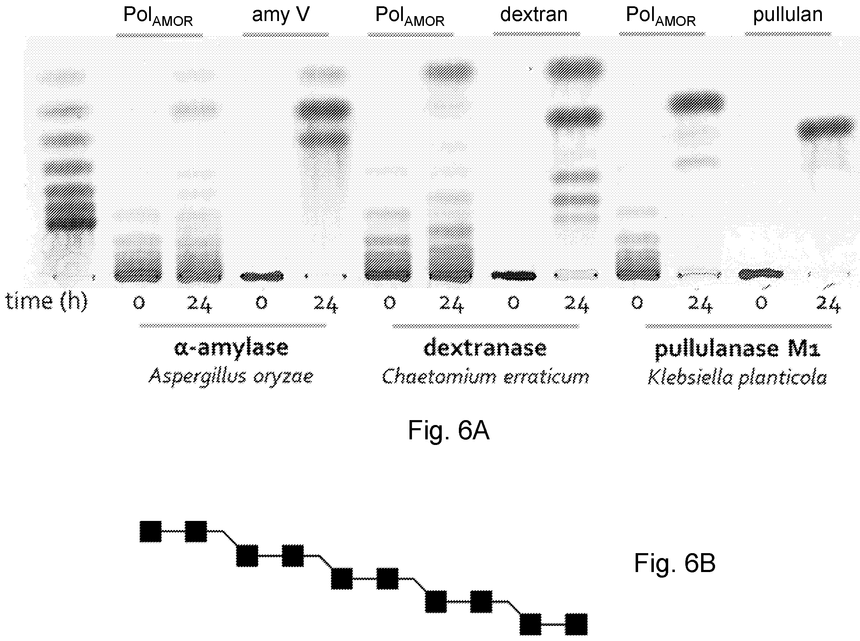

Also disclosed is a linear α-glucan comprising alternating α(l→4) and α(l→6) glucosidic linkages obtainable or obtained by a method according to the invention. The linear α-glucan may comprise alternating α(l→4) and a(l→6) glucosidic linkages and having no α(1→4,6) branching points, less than 0.5% consecutive α(l→6) glucosidic linkages and less than 0.5% consecutive α(l→4) glucosidic linkages. See for example Figure 6B.

Also disclosed is a food item comprising a linear α-glucan according to the invention. The food item can be selected from the group consisting of a dairy product, a dairy analogue, baby or infant formula, bakery product, bakery dough, confectionery product, cereal bar, candy bar, pasta product, noodle product, liquid drink, sport drink, beverage and ice cream.

In one embodiment, the invention provides a baked food product obtained by baking a farinaceous dough comprising a linear α-glucan comprising alternating α(1→4) and α(l→6) glucosidic linkages and having no α(1→4,6) branching points, less than 0.5% consecutive α(l→6) glucosidic linkages and less than 0.5% consecutive α(l→4) glucosidic linkages.

### LEGEND TO THE FIGURES

Fig. 1 Sequence 1 showing the amino acid sequence of GtfC Rv738AMOR representing truncated [amino acids 33 - 738] of >AKM18207 Glucosyltransferase-SI precursor *[Geobacillus* sp. 12AMOR1;]. Conserved motifs are indicated in bold; catalytic residues in underlined italics.
Fig. 2 Sequence 2 showing the amino acid sequence of full length >AKM18207 Glucosyltransferase-SI precursor *[Geobacillus* sp. 12AMOR1;]. Conserved motifs are indicated in bold; catalytic residues in underlined italics.
Fig. 3. Effect of pH (panel A) or temperature (panel B) on GtfC Rv738AMOR enzyme activity.
Fig. 4. Thermal stability of GtfC Rv738AMOR.demonstrated by the effect of prolonged incubation at 60 °C on enzyme activity.
Fig. 5. Oligosaccharide products synthesized by GtfC Rv738AMOR from maltoheptaose (panels A and C) and amylose V (panels B and D) as substrate. Panels A and B: TLC analysis of reactions 1, no enzyme control; G1, glucose; G2, maltose; G3, maltotriose; G4, maltotetraose; G5, maltopentaose; G6, maltohexaose; G7, maltoheptaose. Panels C and D: HPAEC profiles of the oligosaccharide products formed. Green line = G1-G7 standard.
Fig. 6. (panel A) TLC analysis of the GtfC Rv738AMOR polysaccharide product (PolAMOR) before (0) and after (24 h) treatment with hydrolytic enzymes. Positive controls for the α-amylase, dextranase and pullulanase treatments were amylose V, dextran and pullulan, respectively. Panel (B) Model structure of a linear alpha-glucan product comprising alternating α(1→6)/α(1→4) glycosidic bonds.
Fig. 7. Appearance of the wheat breads prepared from 3 different doughs. 1: Reference (no additives); 2: salt solution; 3: GtfC-type enzyme. Panel A: Top/side view. Panel B: top view. Panel C: close up of crumb structure.
Fig. 8. Texture analysis of the wheat breads after 1 day (M1), 2 days (M2), 3 days (M3) or 6 days (M4) of storage. Panel A: Corrected crumb hardness. Panel B: Springiness. Panel C: Resilience.
Fig. 9. Appearance of the gluten-free breads prepared from 4 different doughs. 1: Reference (no additives); 2: salt solution; 3: GtfC-type enzyme. Panel A: side view. Panel B: cross-section. Panel C: close up of crumb structure.
Fig. 10. Corrected crumb hardness of the gluten-free breads after 1 day (M1), 2 days (M2) or 4 days (M3) of storage.
Fig. 11. Corrected crumb hardness of gluten-free breads containing no (Reference) or different dosages of GtfC-type enzyme directly after preparation and following up to 7 days storage at room temperature (20°C). Mean values (n=5) including SD are indicated.

### EXPERIMENTAL SECTION

### EXAMPLE 1: Cloning of the GtfC gene

Eight primer pairs (Table 1) were used to create polynucleotide expression constructs with N-terminal His tags with different GtfC lengths, one for the full-length GtfC protein (amino acids 33-903) without its putative signal peptide-encoding sequence (http://www.cbs.dtu.dk/services/SignalP/; amino acids 1-32) and seven for different C-terminally truncated variants (amino acids 33-738, 33-739, 33-748, 33-752, 33-753, 33-770, 33-902).

The *GtfC* gene fragments were amplified by PCR from *Geobacillus* sp. 12AMOR1 (DSM 17290) genomic DNA (DSMZ, Germany) with Phusion DNA polymerase and cloned into a modified pET15b vector by ligation-independent cloning (LIC). The constructs were verified by nucleotide sequencing (GATC, Cologne, Germany) and transformed into *E. coli* BL21 Star (DE3).

**Table 1. Primers used for cloning of the GtfC 12AMOR1 gene**

| **Primer** | **Sequence (5'-3')** |
|---|---|
| Fw1AMOR | CAGGGACCCGGTTTGATCAAAAAATATGTGCAC |
| Fw33AMOR | CAGGGACCCGGTTATAGCTCCGGCCCGGAATTG |
| Rv738AMOR | CGAGGAGAAGCCCGGTTACGCCTTATCCTTCGTCGGCA |
| Rv739AMOR | CGAGGAGAAGCCCGGTTACGGCGCCTTATCCTTCGTCG |
| Rv748AMOR | CGAGGAGAAGCCCGGTTACGTCTTCGCCGAATTCCACG |
| Rv752AMOR | CGAGGAGAAGCCCGGTTACCCTTGATAAACCGTCTTCGC |
| Rv753AMOR | CGAGGAGAAGCCCGGTTATTTCCCTTGATAAACCGTC |
| Rv770AMOR | CGAGGAGAAGCCCGGTTAGGTTTTAATCTTAGAGGCAGAG |
| Rv902AMOR | CGAGGAGAAGCCCGGTTATCGGACAGTTACGGCAAAATAC |
| Rv903AMOR | CGAGGAGAAGCCCGGTTATCATCGGACAGTTACGGCAAA |

### EXAMPLE 2: GtfC enzyme expression and purification

Luria Broth medium supplemented with ampicillin (100 mg/L) was inoculated with 1% (v/v) of overnight cultures of *E. coli* BL21 (DE3) with the pET15b-GtfC constructs and grown at 37 °C under shaking (160 rpm). Expression was induced by the addition of 0.1 mM IPTG and incubation was continued for 20 h at 18 °C, 160 rpm. Cells were harvested by centrifugation at 4 °C at 10875 x g for 15 min, washed once with 20 mM Tris.HCl pH 8 + 1 mM CaCl₂. Pellets were stored at -20 °C or immediately used for protein isolation. For this, pellets were resuspended in washing buffer (50 mM Tris.HCl pH 8; 250 mM NaCl; 1 mM CaCl₂; 0.25% (v/v) Triton X; 5 mM β-ME) and broken by sonication (Soniprep, 9.5 mm probe, 12 µm amplitude, 6 cycles of 30 sec on and 60 sec off). During sonication the cell suspensions were kept on ice. Cell lysates were centrifuged at 4 °C at 17226 x g for 15 min and the cell-free extracts were stored at 4 °C. To determine whether the proteins were expressed successfully the CFEs were analyzed by SDS-PAGE (data not shown).

All eight pET15b-GtfC constructs showed expression of a protein of the correct size. The GtfC Rv738AMOR construct (726 AA, 82 kDa) was purified by Ni²⁺-nitrilotriacetic acid (NTA) affinity chromatography. Purity was assessed by SDS-PAGE analysis (data not shown).

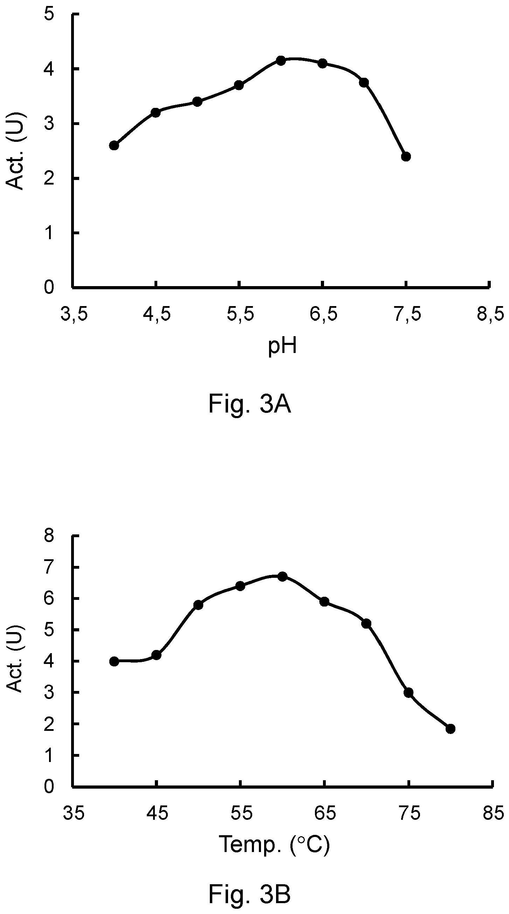

GtfC Rv738AMOR protein was successfully desalted using an Amicon size exclusion column (30 kDa cut off) and desalting buffer (20 mM Tris.HCl pH 8; 1 mM CaCl₂) (Fig. 3). The concentration of pure Rv738AMOR protein (82 kDa; ε₂₈₀ of 104.5 (mM/cm) was determined by the Bradford assay or by measuring the absorbance at 280 nm, using a NanoDrop 2000 spectrophotometer (Isogen Life Science, De Meern, The Netherlands).

### EXAMPLE 3: pH-dependency of GtfC-type enzyme

All enzymatic reactions were performed at 40 °C in 25 mM sodium citrate (pH 6), containing 1 mM CaCl₂ unless mentioned otherwise.

The total activity of the GtfC Rv738AMOR enzyme was determined by measuring the initial rate in the presence of 0.125% (w/v) amylose V (Avebe, Foxhol, The Netherlands) using the amylose-iodine staining method. See e.g.Bai et al., 2015, Applied and environmental microbiology 81 (20), 7223-7232. Enzymatic assays were performed with 50 pg/mL enzyme in reaction buffer at 40 °C.

A decrease in absorbance (660 nm) of the α-glucan-iodine complex resulting from transglycosylation and/or hydrolytic activity was monitored for 6.5 min. The activity expressed in Units was defined as the amount of amylose V (mg) converted by one mg of enzyme per min.

The pH profile and optimum pH of GtfC Rv738AMOR were determined in reaction buffer with different pH values between 4.0 and 7.5 at 40 °C (Fig. 3A). The GtfC Rv738AMOR enzyme showed a maximum activity of 4.1 U at pH 6, and retained more than 80% of this activity over a pH range from 4.5 to 7.

### EXAMPLE 4: Temperature optimum and thermal stability of the GtfC enzyme

The temperature optimum for enzyme activity of GtfC Rv738AMOR was determined using a reaction mixture comprising 50 pg/mL enzyme and 0.125% amylose V at varying temperatures between 40 and 80 °C (Fig. 3B). The activity of GtfC Rv738AMOR increased from 4 U at 40 °C to 6.6 U at 60 °C and gradually decreased to 3 U at 75 °C.

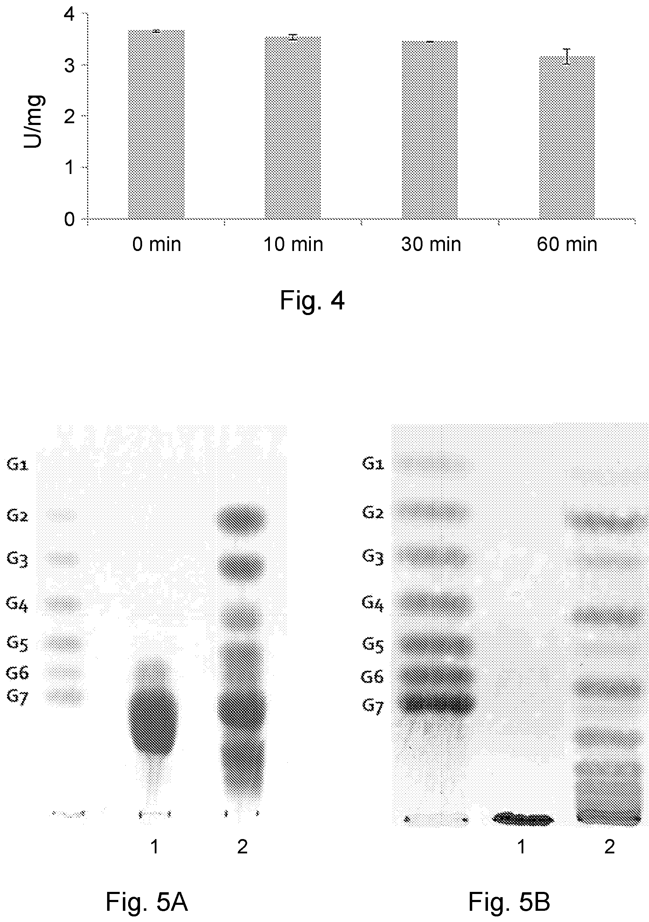

The thermostability of the enzyme was investigated by measuring the residual enzyme activity (initial rate) after incubation at 60 °C for different time periods. For this, 50 pg/mL enzyme was incubated in reaction buffer in the absence of amylose V for 0, 10, 30 and 60 min at 60 °C and then immediately cooled to 4 °C. The residual enzyme activity (initial rate) of the heat-treated enzyme preparations was measured at 40 °C with 0.125% Amylose V. Figure 4 shows that GtfC Rv738AMOR is very stable at 60 °C, showing only a relatively minor (<10%) decrease in activity after one hour incubation at 60 °C. Differential scanning fluorimetry using SYPRO orange as Thermofluor revealed a melting temperature for GtfC Rv738AMOR of 69 °C, thus confirming a very good thermostability.

### EXAMPLE 5: Reaction and substrate specificity of GtfC enzyme

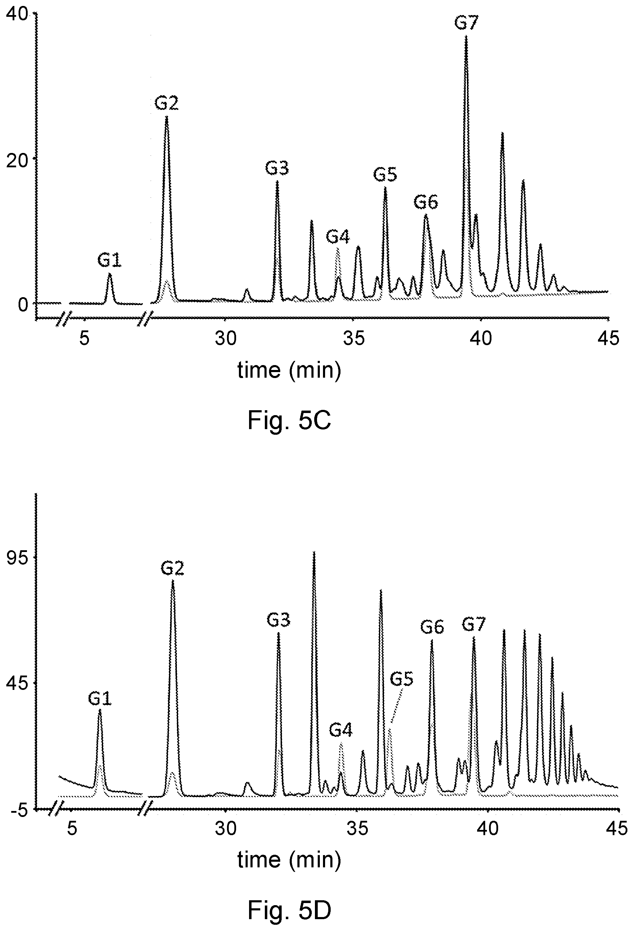

The reaction specificity of GtfC Rv738AMOR was investigated by incubating the enzyme (40 µg/mL) with maltoheptaose (G7; 25 mM) or amylose V (0.5% w/v) for 24 h. TLC and HPAEC analysis of the products formed from G7 revealed that GtfC Rv738AMOR has disproportionating activity, synthesizing both shorter and longer oligosaccharides (Fig. 5). When acting on amylose V, GtfC Rv738AMOR synthesized oligo/poly-saccharides of various sizes.

Surprisingly, GtfC Rv738AMOR released some maltose, and very little glucose from G7 and amylose V. Furthermore, the HPAEC elution profiles revealed that GtfC Rv738AMOR had formed compounds with elution times distinct from linear α(1→4) oligosaccharides, demonstrating that the enzyme forms glycosidic linkages distinct from α(1→4) glycosidic bonds (Fig. 5 C and D).

The oligosaccharide product obtained from amylose V upon GtfC Rv738AMOR incubation was analyzed further. First, the reaction product was dialyzed against MilliQ water, using a 3.5 kDa snake skin tubing (ThermoScientific) and the product was subsequently lyophilized.

The product obtained (3 mg/mL, 50 mM sodium acetate, pH 5.0) was treated with three different types of α-glucan hydrolyzing enzymes. Subsequent TLC analysis revealed that the product is largely resistant to the action of the endo-α(1→4)-hydrolase action of α-amylase *(Aspergillus oryzae)* and the endo-α(1→6)-hydrolase activity of dextranase *(Chaetomium erraticum)* (Fig. 6A).

In contrast, the product was essentially completely hydrolyzed by the pullulanase M1 *(Klebsiella planticola*) yielding maltose. Pullulanase M1 hydrolyses(α(1→6) linkages that are present in linear and branched α-glucan polymers such as pullulan and amylopectin. Accordingly, this result indicates that the product of GtfC Rv738AMOR is composed of glucose moieties connected via alternating α(1→6)/α(1→4) glycosidic linkages.

Subsequent methylation analysis revealed that the products consist of terminal (8.6%), 4-substituted (48.4%) and 6-substituted (43.0%) glucopyranoside moieties, and that there are no 4,6-substituted glucopyranose moieties (branching points).

Together, these data demonstrate that GtfC Rv738AMOR cleaves off maltose units from the nonreducing end of (α1→4) glucan chains. The cleaved off maltose unit is then transferred to the nonreducing end of another chain, forming an α(1→6) glycosidic linkage. This process is repeated and results in linear chains of alternating α(1→4)/α(1→6) glycosidic linkages. See Figure 6B for a schematic representation. In addition, the GtfC Rv738AMOR enzyme has some hydrolyzing side activity resulting in the formation of maltose.

### EXAMPLE 6: Preparation and characterization of wheat bread

This example demonstrates the beneficial effect of incorporating a GtfC-type enzyme in a wheat flour dough.

**Table 2 Ingredients used for wheat bread.**

| **Product** | **Commercial name** | **Supplier** |
|---|---|---|
| Wheat flour | Amerikaanse patentbloem | Koopmans |
| Instant yeast | Instant yeast | Bruggeman |
| Sugar (fine) | Sugar (fine) | Suikerunie |
| Sodium chloride | Salt | Jozo |
| Sunflower oil | Sunflower oil | Reddy |

**Table 3 shows the relevant parameters of the GTFC enzyme used.**

| Enzyme | Recomb. Source | pH optim um | Temperature optimum | Inactivation temperature (in solution) | Activity level | Dry matter |
|---|---|---|---|---|---|---|
| GTFC | E.coli | ~6 | ~60°C | ~85°C | 7,000 U/g | 1.77% |

Different doughs were prepared according to the following recipes (Table 4). The dosage of GTFC enzyme was 430 ppm (dry enzyme on total composition).

Because of the fact that the GTFC enzyme was present in a solution (1.77% w/w) also comprising salts and buffers (~1%), a salt solution without the enzyme was included in a further control bread. The salt solution was prepared by dialysis of the enzyme solution, and taking the inside of the dialysis membrane as enzyme solution and the outside as control. In this way, possible effects of the salts and buffers could be excluded.

**Table 4**

| | Reference | | Salt solution | | *0.043% enzyme (d.m.)* | |
|---|---|---|---|---|---|---|
| | | | | | *7.08 U*/*g starch* | |
| | | | | | *7080 U*/*kg starch* GTFC-enzyme | |
| | 1 | | 2 | | 3 | |
| | G | % | G | % | G | % |
| **Ingredient** | **Recipe** | **Recipe** | **Recipe** | **Recipe** | **Recipe** | **Recipe** |
| Wheat flour | 1243 | 60.6% | 1243 | 60.6% | 1243 | 60.6% |
| Water | 746 | 36.4% | 703 | 34.3% | 703 | 34.3% |
| Sugar (fine) | 12 | 0.6% | 12 | 0.6% | 12 | 0.6% |
| Instant yeast | 19 | 0.9% | 19 | 0.9% | 19 | 0.9% |
| Oil (sunflower oil) | 12 | 0.6% | 12 | 0.6% | 12 | 0.6% |
| Salt | 17 | 0.8% | 17 | 0.8% | 17 | 0.8% |
| GTFC-enzyme (1.77% d.m.) | | | 44.1 | 2.2% | | |
| Membrane/salt solution (1% d.m.) | | | | | 44.1 | 2.2% |
| | 2050 | 100% | 2051 | 100% | 2051 | 100% |

Breads were prepared using the following steps with the use of a Diosna spiral mixer:
1. Mix the dry ingredients (except yeast) into a homogeneous mixture
2. Add yeast to the mixture and mix into a homogeneous mixture
3. Mix the GTFC solution or salt solution with water (40°C) into a homogenous mixture
4. Add the water (40°C), GTFC solution (40°C) or salt solution (40°C) to the mixture and mix it into a homogeneous mixture (4 min. at slow speed, add oil and mix for 1 min. more)
5. Mix it for approx. 8 minutes into a solid dough at high speed
6. Divide in pieces of 220 gram and shape
7. Bulk fermentation: 60 minutes at 26-28°C | RH: 80-90 %
8. Press the gas out of the dough and shape again
9. Final fermentation: 50 minutes at 26-28°C | RH: 80-90 %
10. Baking profile: initial temperature 220°C | baking temperature 200°C during 50 minutes with steam injection (2x) | after 30 minutes the valve was opened

### Bread Texture analysis

Texture profiles of the bread crumbs were obtained with the help of a Shimadzu Texture Analyser after one day (M1), two days (M2), three days (M3) and six days (M4) of storage at room temperature to monitor the staling rate. Four cylindrical pieces with a thickness of 2 cm and a diameter of 4cm, derived from the center of the were two times compressed for a distance of 3 mm with a cylindrical probe (P75; diameter 3.5 cm) at a speed of 1 mm/s. In this manner, 3 relevant parameters were determined.
- Hardness: parameter for the hardness or softness of the crumb determined with the maximum peak force (gram-force) during the first compression cycle. Hardness was corrected by the weight of each sample.
- Resilience: how well a product fights to regain its original height, calculated with the following formula: $Resilience \left(%\right) = \frac{Surface of decompression curve \left(Area 4\right)}{Surface of compression curve \left(Area 3\right)} * 100$
- Springiness: how well a product physically springs back after it has been deformed during the first compression and has been allowed to wait for the target wait time between the compressions, calculated with the following formula: $Springiness \left(%\right) = \frac{\left(\frac{Surface of 2 nd compression curve \left(Area 5\right)}{Max . force 2 nd compression}\right)}{\left(\frac{Surface of 1 st compression curve \left(Area 3\right)}{Max . force 1 st compression}\right)} ∗ 100$

### Appearance, specific volume, baking loss and dough pH

Specific volume was determined by weighing the cylindrical pieces used for texture analysis, followed by calculating the specific volume based on the height (h= 2 cm) and radius (r= 2cm) of the cylindrical pieces according to the following formula: *V = π* * *r*² * *h.*

Appearance of the breads was captured by taking a picture of the loaves after baking, which also shows the visual volume that was developed during the process. Baking loss was determined by subtracting the weight of the dough (300 g) from the weight of the breads after baking. Furthermore, breads were judged in a sensory evaluation by manually pressing the dough structure and observing the moistness of the crumbs.

### Results

### Appearance, (specific) volume, baking loss and dough pH

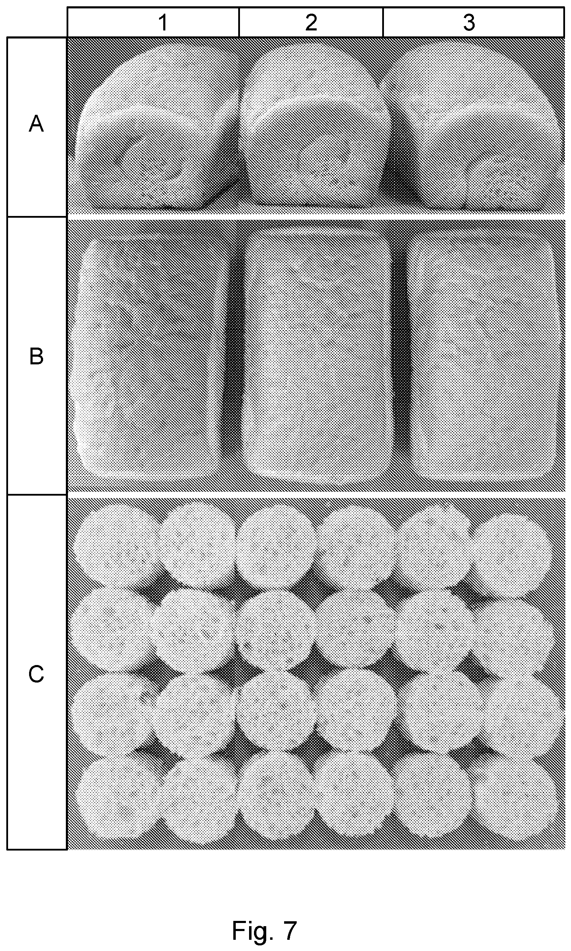

Figure 7 shows the appearance of the breads and crumbs. No major differences in appearance and crumb structure are observed. This also broadly counts for loaf volume, specific crumb volume, baking loss and dough pH. Dough pH of the recipes was 5.4, which is near the optimum of the GTFC-enzyme.

**Table 5**

| | 1. Reference | 2. Salt solution | 3. GTFC |
|---|---|---|---|
| Average weight bread (g) | 179_{± 1} | 180 _{± 1} | 181_{± 1} |
| Average weight cube (g) | 5.6_{± 0,2} | 6.3 _{± 0,1} | 5.8 _{± 0,3} |
| Baking loss (%) | 19% _{±0,3} | 18% _{± 0,4} | 18% _{± 0,3} |
| Specific crumb volume (g/ml) | 0.22_{± 0,01} | 0.23_{± 0,01} | 0.23_{± 0,01} |
| Loaf volume (ml) | 822_{± 19} | 815 _{± 6} | 811 _{±22} |
| pH dough | 5.4 | 5.4 | 5.4 |

### Texture analysis

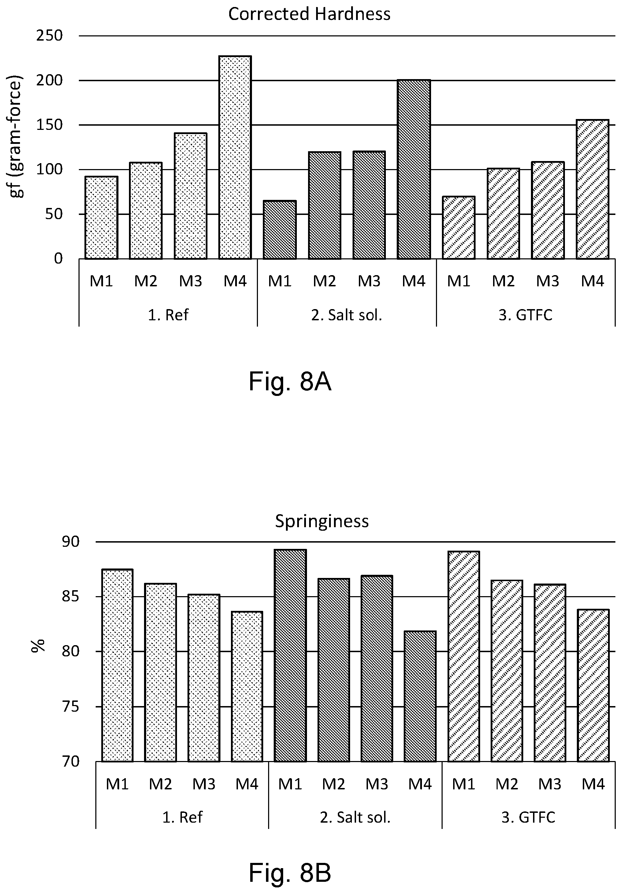

Figure 8A shows the corrected crumb hardness during storage. In all recipes an increase in corrected hardness is noticeable, which is caused by bread staling. During the first 2 days (M1 and M2), all recipes show a comparable corrected hardness while after 3 days of storage (M3) the breads containing the GTFC-enzyme show a slightly lower corrected crumb hardness compared to the 2 control recipes. This difference becomes significant after 6 days of storage (M4) which results in a lower absolute and relative crumb corrected hardness increase of the recipes prepared with the GTFC-enzyme during a storage time of 6 days (Table 6).

Table 6: Increase in relative corrected hardness (% C. hardness increase) and absolute corrected hardness increase (abs. C. hardness increase) during storage between M1-M2: 1 day and 2 days of storage, between M2-M3: 2 and 3 days of storage, between M3-M4: 3 and 6 days of storage and M1-M4: 1 day and 6 days of storage.

**Table 6**

| | 1. Reference | | | | 2. Salt solution | | | | 3. GTFC | | | |
|---|---|---|---|---|---|---|---|---|---|---|---|---|
| | M1-M2 | M2-M3 | M3-M4 | M1-M4 | M1-M2 | M2-M3 | M3-M4 | M1-M4 | M1-M2 | M2-M3 | M3-M4 | M1-M4 |
| % C. Hardness increase | 17% | 31% | 62% | 146 % | 84% | 0.4 % | 67% | 209 % | 45% | 7% | 44% | 124 % |
| Abs. C. hardness increase (gf) | 16 | 33 | 87 | 135 | 55 | 1 | 80 | 136 | 31 | 7 | 47 | 86 |

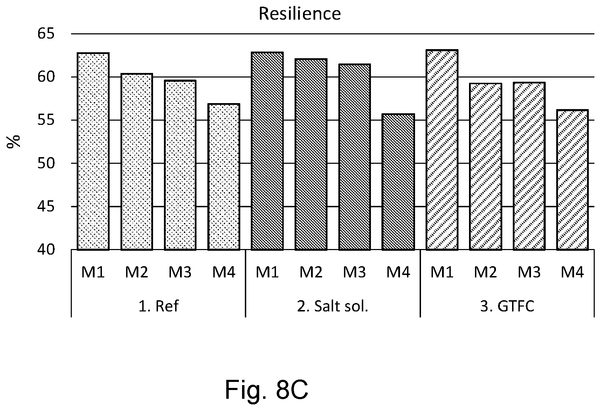

Figure 8B shows the springiness and Figure 8C shows the resilience of the bread crumbs during storage. All recipes show a similar trend in degradation of elasticity (springiness & resilience) which is related to the staling process. Despite the lower softness of the GTFC-supplemented wheat bread, the resilience and springiness are on the same level as the reference breads.

### EXAMPLE 7: Preparation and characterization of gluten-free bread

This example demonstrates the effect on the staling rate of a GTFC-like enzyme in gluten-free (GF-)bread.

GF-bread is more sensitive for staling in comparison to wheat based breads. Moreover, starch gelatinization takes place at an earlier stage during baking in GF-bread because it contains often potato starch and higher levels of water, as opposed to wheat based breads containing wheat starch. Table 7 shows the general ingredients and Table 8 shows the potato-derived ingredients used for the experiments.

**Table 7 Ingredients used for gluten-free bread**

| Product | Commercial name | Supplier |
|---|---|---|
| Sugar (coarse) | Sugar (coarse) | Suikerunie |
| Instant yeast | Instant yeast | Dr. Oetker |
| Brown rice flour (78% w/w starch) | Remyflo C2000 | Beneo |
| Inulin | Frutafit HD | Cosun |
| Psyllium husk fiber | Psyllium husk powder 95% | Roeper |
| Sodium chloride | Salt | Jozo |
| Sunflower oil | Sunflower oil | Reddy |
| Baking spray | Baking spray | Dr. Oetker |
| Sodium bicarbonate | Sodium bicarbonate | Beko |
| Carboxymethylcellulose (CMC) | Cekol 30.000 | CP Kelco |

**Table 8: potato-derived ingredients (all from Avebe NV, The Netherlands)**

| **Product** | **Commercial name** |
|---|---|
| Native potato starch (79.5% w/w starch) | Native potato starch |
| Fine granulated potato starch (79.5% w/w starch) | Selectamyl D20 |
| Extruded instant potato starch (89% w/w starch) | Paselli WA5 |
| Potato fiber (insoluble) (20% w/w starch) | Paselli FP |
| Potato protein | Solanic 300 |

Table 9 shows the recipes of the various GF-doughs, including a reference dough without enzyme, and a reference dough without enzyme but with the salt solution The dosage of GTFC-enzyme was set on 350 ppm on total composition.

**Table 9**

| | | | | | *0.035% enzyme (d. m.)* | |
|---|---|---|---|---|---|---|
| | | | | | *9.608 U*/*g starch* | |
| | | | | | *9608 U*/*kg starch* | |
| | Reference | | Salt solution | | GTFC-enzyme | |
| | 1 | | 2 | | 3 | |
| | % | G | % | G | % | G |
| **Ingredient** | **Recipe** | **Recipe** | **Recipe** | **Recipe** | **Recipe** | **Recipe** |
| Solanic 300 | 4.0% | 40 | 4.0% | 40 | 4.0% | 40 |
| Paselli FP | 3.0% | 30 | 3.0% | 30 | 3.0% | 30 |
| Selectamyl D20 | 21.9% | 219 | 21.9% | 219 | 21.9% | 219 |
| Potato starch | 4.0% | 40 | 4.0% | 40 | 4.0% | 40 |
| Paselli WA5 | 0.5% | 5 | 0.5% | 5 | 0.5% | 5 |
| Water | 51.4% | 514 | 49.4% | 494 | 49.4% | 494 |
| Sunflower oil | 4.0% | 40 | 4.0% | 40 | 4.0% | 40 |
| Inulin | 1.0% | 10 | 1.0% | 10 | 1.0% | 10 |
| Psyllium husk | 0.4% | 4 | 0.4% | 4 | 0.4% | 4 |
| Brown rice flour | 5.0% | 50 | 5.0% | 50 | 5.0% | 50 |
| CMC | 1.0% | 10 | 1.0% | 10 | 1.0% | 10 |
| Sugar | 1.7% | 17 | 1.7% | 17 | 1.7% | 17 |
| Instant yeast | 1.0% | 10 | 1.0% | 10 | 1.0% | 10 |
| Salt | 0.8% | 8 | 0.8% | 8 | 0.8% | 8 |
| Sodium bicarbonate | 0.4% | 4 | 0.4% | 4 | 0.4% | 4 |
| GTFC-enzyme (1.77% d.m.) | | | | | 2.0% | 20 |
| Membrane/salt solution (1% d.m.) | | | 2.0% | 20 | | |
| Total | 100% | 1000 | 100% | 1000 | 100% | 1000 |

Gluten-free breads were prepared using the following steps with the use of a Hobart mixer with a flat beater:
1. Mix the dry ingredients (except yeast) into a homogeneous mixture by hand
2. Add oil to the bowl and mix it in the Hobart kneader for 1 minute at gear 1
3. Add yeast to the mixture and mix again 1 minute at gear 1
4. Mix the GTFC solution or salt solution with water (39 °C) into a homogenous mixture
5. Add the water (39°C), GTFC solution (39 C) or salt solution (39°C ) to the mixture, while mixing at gear 1 for 2 minutes
6. Scrap the batter from the bottom by hand and mix it for 2 minutes more at gear 1 and 6 minutes at gear 2
7. Divide the dough into oil sprayed baking tins in portions of 300 grams
8. Final proofing: 30 minutes at 30 °C/90% RH
9. Baking profile: appr. 30 minutes at 235 °C upper and 245 °C bottom temperature
   - With appr. 4 seconds (2 shots) steam injection
   - After 20 minutes the valve is opened

Same analytical methods were applied as in Example 6, although texture profiles were determined at different moments; after one day (M1), 2 days (M2) and 4 days (M3) of storage.

### Appearance, specific volume, baking loss and dough pH

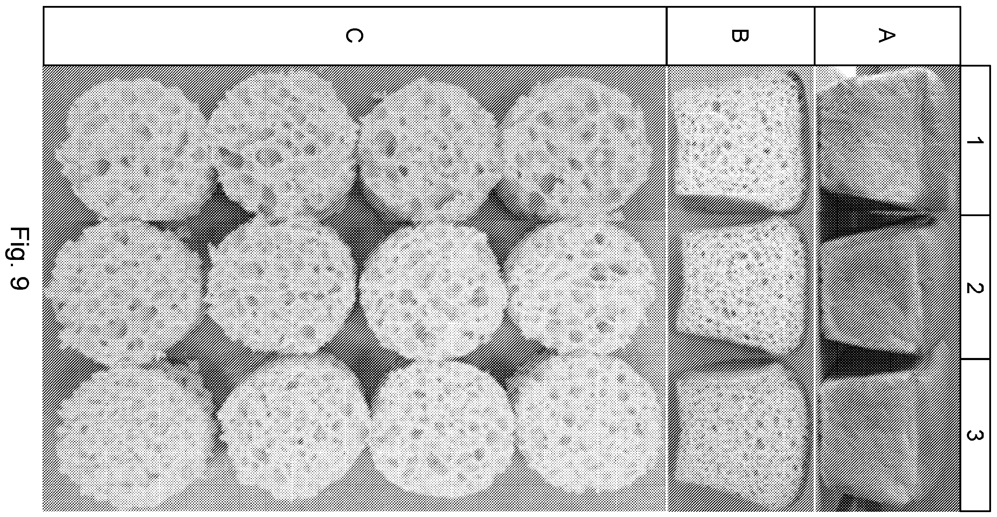

Figure 9 shows the appearance of the breads and crumbs. No major differences were observed between the breads. Although the crumb structure of the recipe with GTFC enzyme seems slightly finer compared to the other recipes.

The pH value of the GF-doughs was comparable, and at an optimal performance level for the GTFC enzyme.

**Table 10**

| | 1.Reference | 2. Salt solution | 3. GTFC |
|---|---|---|---|
| Average weight bread (g) | 255 | 256 | 256 |
| Average weight cube (g) | 6.93 | 7.13 | 7.35 |
| Baking loss (%) | -16% | -15% | -15% |
| Specific volume (g/ml) | 0.28 | 0.29 | 0.29 |
| pH dough | 5.93 | 5.92 | 5.95 |

### Texture analysis

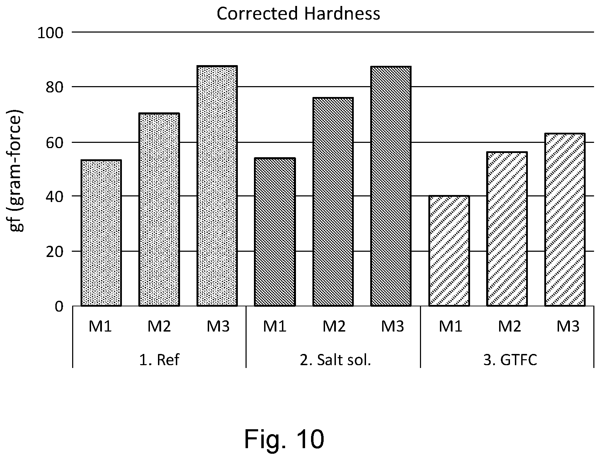

Figure 10 shows the corrected crumb hardness during storage of 4 days. The bread with GTFC enzyme shows at each measuring moment M1, M2 and M3 (1, 2 and 4 days after baking, respectively) a significantly lower corrected hardness compared to the other recipes. This softness was also visually noticeable after manually compressing each sample. Both reference breads, including the one with salt solution, show a similar initial corrected hardness and firming rate during storage, which proves that the salt solution of the enzyme does not influence the crumb hardness.

### EXAMPLE 8: Dose-dependent anti-staling effect

This example shows that incorporating a GtfC-type enzyme has a dose-dependent effect on the staling rate of gluten-free bread.

| Enzyme | Recomb. Source | pH optimum | Temperature optimum | Inactivation temperature (in solution) | Activity level | Dry matter |
|---|---|---|---|---|---|---|
| GTFC | E.coli | ~6 | ~60°C | ~85°C | 3650 U/g | 1.09% |

Five doughs were prepared using essentially the same ingredients as described in Example 7. These included a reference dough without enzyme, and 4 doughs comprising different dosages: 0.069%; 0.035%; 0.0069% or 0.00069% enzyme (d.m.) of AMOR1 GtfC-type enzyme. This corresponded to about 100%, 50%, 10% and 1% of the enzyme dosage used in Example 7. See table 11 for the recipes.

The same dough and bread preparation method was applied as described as in Example 7. The same texture measuring method was applied as described in Example 6, although the texture of the resulting breads was in this case analyzed at day zero (3 hours after baking), and after 1, 2, 3, 4 and 7 days storage at room temperature. Next to that, the compression distance of the probe was 2.8 mm instead of 3 mm, compared to the compression distance applied in Example 6 and 7. The total number of samples tested at each measuring moment was 5.

| Table 11 | | | *0.069% enzyme (d.m.)* | | *0.035% enzyme (d.m.)* | | *0.0069% enzyme (d.m.)* | | *0.00069% enzyme (d.m.)* | |
|---|---|---|---|---|---|---|---|---|---|---|
| | | | *9.604 U*/*g starch* | | *4.802 U*/*g starch* | | *0.9604 U*/*g starch* | | *0.09604 U*/*g starch* | |
| | | | *9604 U*/*kg starch* | | *4802 U*/*kg starch* | | *960.4 U*/*kg starch* | | *96.04 U*/*kg starch* | |
| | Reference | | GTFC-enzyme | | GTFC-enzyme | | GTFC-enzyme | | GTFC-enzyme | |
| | 1 | | 2 | | 3 | | 4 | | 5 | |
| | **%** | **G** | **%** | **G** | **%** | **G** | **%** | **G** | **%** | **G** |
| **Ingredient** | **Recipe** | **Recipe** | **Recipe** | **Recipe** | **Recipe** | **Recipe** | **Recipe** | **Recipe** | **Recipe** | **Recipe** |
| Solanic 300 | 4.0% | 100 | 4.0% | 100 | 4.0% | 100 | 4.0% | 100 | 4.0% | 100 |
| Paselli FP | 3.0% | 75 | 3.0% | 75 | 3.0% | 75 | 3.0% | 75 | 3.0% | 75 |
| Selectamyl D20 | 21.9% | 546 | 21.9% | 546 | 21.9% | 546 | 21.9% | 546 | 21.9% | 546 |
| Potato starch | 4.0% | 100 | 4.0% | 100 | 4.0% | 100 | 4.0% | 100 | 4.0% | 100 |
| Paselli WA5 | 0.5% | 13 | 0.5% | 13 | 0.5% | 13 | 0.5% | 13 | 0.5% | 13 |
| Water | 51.4% | 1284 | 45.1% | 1125 | 48.2% | 1204 | 50.8% | 1268 | 51.3% | 1282 |
| Sunflower oil | 4.0% | 100 | 4.0% | 100 | 4.0% | 100 | 4.0% | 100 | 4.0% | 100 |
| Psyllium husk | 0.4% | 10 | 0.4% | 10 | 0.4% | 10 | 0.4% | 10 | 0.4% | 10 |
| Brown rice flour | 6.0% | 150 | 6.0% | 150 | 6.0% | 150 | 6.0% | 150 | 6.0% | 150 |
| CMC | 1.0% | 25 | 1.0% | 25 | 1.0% | 25 | 1.0% | 25 | 1.0% | 25 |
| Sugar | 1.7% | 43 | 1.7% | 43 | 1.7% | 43 | 1.7% | 43 | 1.7% | 43 |
| Instant yeast | 1.0% | 25 | 1.0% | 25 | 1.0% | 25 | 1.0% | 25 | 1.0% | 25 |
| Salt | 0.8% | 20 | 0.8% | 20 | 0.8% | 20 | 0.8% | 20 | 0.8% | 20 |
| Sodium bicarbonate | 0.4% | 10 | 0.4% | 10 | 0.4% | 10 | 0.4% | 10 | 0.4% | 10 |
| GTFC-enzyme (1.09% d.m.) | | | 6.3% | 158.6 | 3.2% | 79.3 | 0.63% | 15.9 | 0.063% | 1.59 |
| Total | 100% | 2500 | 100% | 2500 | 100% | 2500 | 100% | 2500 | 100% | 2500 |

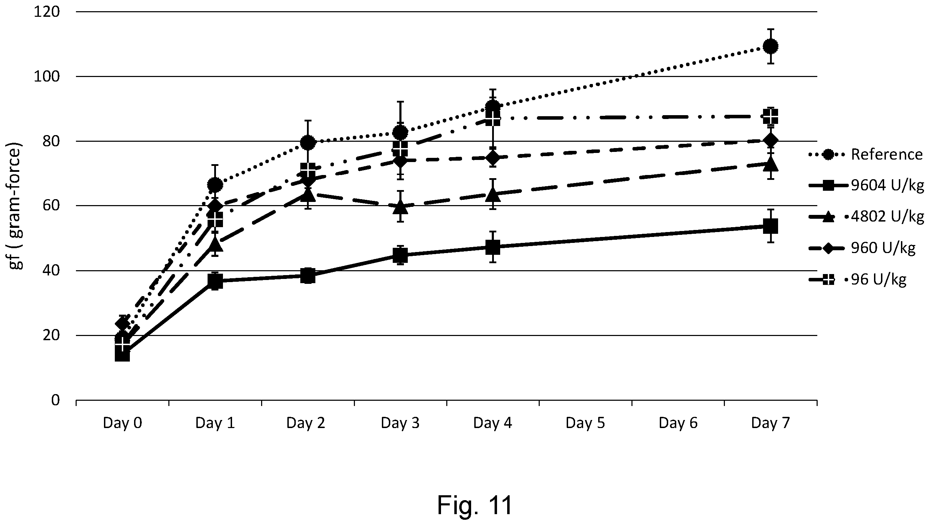

Figure 11 shows the corrected crumb hardness during Storage at room temperature. It confirms the results of Example 7 that including the GTFC enzyme results in significantly softer breads, and that the level of hardness reduction is dose-dependent. For the low and intermediate enzyme dosages, beneficial effects relative to the breads without enzyme are most evident after a few (>2) days of storage, whereas at higher dosages the beneficial effects can be observed as early as immediately after baking, which effect continues to increase during prolonged storage.

## Claims

1. A method of preparing a baked food product by baking a farinaceous dough, comprising incorporating into the dough a GtfC-type 4,6-α-glucanotransferase enzyme, wherein the GtfC-type enzyme (i) is capable of transferring a maltose moiety from a polysaccharide or oligosaccharide substrate to the non-reducing end of an oligosaccharide acceptor via a new α(1→6) linkage without forming α(1→ 4,6) branching points, and (ii) has an activity optimum in the temperature range of 50-70°C determined at pH 6 with amylose V as substrate.

2. A farinaceous dough comprising a GtfC-type 4,6-α-glucanotransferase enzyme, wherein the GtfC-type enzyme (i) is capable of transferring a maltose moiety from a polysaccharide or oligosaccharide substrate to the non-reducing end of an oligosaccharide acceptor via a new α(1→6) linkage without forming α(1→ 4,6) branching points, and (ii) has an activity optimum in the temperature range of 50-70°C determined at pH 6 with amylose V as substrate.

3. The method or dough according to claim 1 or 2, wherein the GtfC-type enzyme has an activity optimum in the temperature range of 55-65°C.

4. The method or dough according to any one of the preceding claims,
wherein the GtfC-type enzyme has at least 80% residual 4,6-α-glucanotransferase activity after incubating the enzyme in a 25 mM sodium citrate buffer containing 1 mM CaCl₂ at a temperature of 60°C at pH 6 for 60 min.

5. The method or dough according to any one of the preceding claims,
wherein the GtfC-type enzyme has an activity optimum in the pH range of 4 to 7.5, preferably 5.0 to 7.0, more preferably 5.5-6.5, in a 25 mM sodium citrate buffer containing 1 mM CaCl₂ at a temperature of 40°C with amylose V as substrate.

6. The method or dough according to any one of the preceding claims,
wherein the GtfC-type enzyme is incorporated in the dough in an amount of 100 to 20,000 U per kg of the total weight of starch in the dough, preferably in an amount of 500 to 15,000 U/kg starch, wherein the activity expressed in Units is defined as the amount of amylose V (mg) converted by one mg of enzyme per min determined in a 25 mM sodium citrate buffer containing 1 mM CaCl₂ at a temperature of 60°C at pH 6 with amylose V as substrate.

7. The method or dough according to any one of the preceding claims,
wherein the GtfC-type enzyme is a polypeptide having the GenBank accession number AKM18207, or a functional fragment thereof having the desired GtfC-type 4,6-α-glucanotransferase activity.

8. The method or dough according to any one of the preceding claims,
wherein the GtfC-type enzyme is a polypeptide comprising an amino acid sequence having at least 80% identity to Sequence 1 (Fig. 1), preferably comprising an amino acid sequence having at least 80% identity to Sequence 2 (Fig. 2).

9. The method or dough according to claim 8, wherein the polypeptide comprises the conserved motifs I, II, III and IV of the GTFC-like members of the GH70 family, wherein the motifs have the following amino acid sequences:
Motif I: [M/E/L]DLVPNQ, preferably LDLVPNQ
Motif II: GFRIDAA[S/G/T]H[Y/F]D, preferably GFRIDAATHFD
Motif III: HLSYIESY[K/S/Q/T][S/N][E/V/A/K], preferably HLSYIESYTSK
Motif IV: FV[N/T]NHDQEKNR[V/I]N[Q/N/T], preferably FVNNHDQEKNRVNT.

10. The method or dough according to any one of the preceding claims,
wherein the dough is prepared by combining flour, water, yeast, GtfC-type 4,6-α-glucanotransferase enzyme and optionally other bakery ingredients.

11. The method or dough according to any one of the preceding claims,
wherein the dough is a wheat flour dough.

12. The method or dough according to any one of claims 1-11, wherein the dough is a gluten-free or gluten-reduced dough, preferably wherein the dough comprises native potato protein in an amount of 0.2 to 8 w/w % on total dough.

13. A baked food product obtainable by a method according to any one of claims 1, 3-12 or by baking a dough according to any one of claims 2-12.

14. A baked food product obtained by baking a farinaceous dough comprising a linear α-glucan comprising alternating α(1→6)/α(1→4) glycosidic bonds and having no α(1→4,6) branching points, less than 0.5% consecutive α(1→6) glycosidic bonds and less than 0.5% consecutive α(1→4) glycosidic bonds.

15. A method to increase the shelf-life of a baked food product, preferably a bread, comprising incorporating into a farinaceous bread dough a GtfC-type 4,6-α-glucanotransferase enzyme which is capable of transferring a maltose moiety from a polysaccharide or oligosaccharide substrate to the non-reducing end of an oligosaccharide acceptor via a new α(1→6) linkage without forming α(1→4,6) branching points, and wherein the GtfC-type enzyme has an optimum temperature in the range of 50-70°C determined at pH 6 with amylose V as substrate, and baking the dough.

16. The use of a GtfC-type 4,6-α-glucanotransferase enzyme as an anti-staling agent in a baked dough product, wherein said GtfC-type 4,6-α-glucanotransferase enzyme is capable of transferring a maltose moiety from a polysaccharide or oligosaccharide substrate to the non-reducing end of an oligosaccharide acceptor via a new α(1→6) linkage without forming α(1→4,6) branching points, and wherein the GtfC-type enzyme has an optimum temperature in the range of 50-70°C determined at pH 6 with amylose V as substrate.

17. The use of a GtfC-type 4,6-α-glucanotransferase enzyme to improve or preserve at least one property of a baked dough product, preferably to improve or preserve one or more of loaf volume, crumb structure, crumb resilience, crumb springiness, dough strength, dough stability and crumb softness, wherein said GtfC-type 4,6-α-glucanotransferase enzyme is capable of transferring a maltose moiety from the polysaccharide or oligosaccharide substrate to the non-reducing end of an oligosaccharide acceptor via a new α(1→6) linkage without forming α(1→4,6) branching points, and wherein the GtfC-type enzyme has an optimum temperature in the range of 50-70°C determined at pH 6 with amylose V as substrate.

## Patentansprüche

1. Verfahren zur Herstellung eines gebackenen Lebensmittelprodukts durch Backen eines mehlhaltigen Teigs, umfassend das Einbringen eines GtfC-Typ 4,6-α-Glucanotransferase-Enzyms in den Teig, wobei das GtfC-Typ Enzym (i) in der Lage ist, eine Maltoseeinheit von einem Polysaccharid- oder Oligosaccharidsubstrat auf das nichtreduzierende Ende eines Oligosaccharidakzeptors über eine neue α(1→6)-Bindung zu übertragen, ohne α(1→4,6)-Verzweigungspunkte zu bilden, und (ii) ein Aktivitätsoptimum im Temperaturbereich von 50-70 °C hat, bestimmt bei pH 6 mit Amylose V als Substrat.

2. Ein mehlhaltiger Teig, umfassend ein GtfC-Typ 4,6-α-Glucanotransferase-Enzym, wobei das GtfC-Typ Enzym (i) in der Lage ist, eine Maltoseeinheit von einem Polysaccharid- oder Oligosaccharidsubstrat auf das nichtreduzierende Ende eines Oligosaccharidakzeptors über eine neue α(1→6)-Bindung zu übertragen, ohne α(1→4,6)-Verzweigungspunkte zu bilden, und (ii) ein Aktivitätsoptimum im Temperaturbereich von 50-70 °C hat, bestimmt bei pH 6 mit Amylose V als Substrat.

3. Verfahren oder Teig nach Anspruch 1 oder 2, wobei das GtfC-Typ Enzym ein Aktivitätsoptimum im Temperaturbereich von 55-65 °C hat.

4. Verfahren oder Teig nach einem der vorhergehenden Ansprüche, wobei das GtfC-Typ Enzym mindestens 80 % Rest-4,6-α-Glucanotransferase-Aktivität hat, nach Inkubation des Enzyms in einem 25-mM-Natriumcitratpuffer, enthaltend 1 mM CaCl₂, bei einer Temperatur von 60 °C bei pH 6 für 60 min.

5. Verfahren oder Teig nach einem der vorhergehenden Ansprüche, wobei das GtfC-Typ Enzym ein Aktivitätsoptimum im pH-Bereich von 4 bis 7,5, vorzugsweise 5,0 bis 7,0, bevorzugter 5,5-6,5 hat, in einem 25-mM-Natriumcitratpuffer, enthaltend 1 mM CaCl2, bei einer Temperatur von 40 °C mit Amylose V als Substrat.

6. Verfahren oder Teig nach einem der vorhergehenden Ansprüche, wobei das GtfC-Typ Enzym in den Teig in einer Menge von 100 bis 20.000 U pro kg des Gesamtgewichts an Stärke im Teig, vorzugsweise in einer Menge von 500 bis 15.000 U/kg Stärke, eingebracht wird, wobei die Aktivität ausgedrückt als Einheiten als die Menge an Amylose V (mg) definiert ist, umgesetzt von einem mg Enzym pro min, bestimmt in einem 25-mM-Natriumcitratpuffer, enthaltend 1 mM CaCl₂, bei einer Temperatur von 60 °C, bei pH 6 mit Amylose V als Substrat.

7. Verfahren oder Teig nach einem der vorhergehenden Ansprüche, wobei das GtfC-Typ Enzym ein Polypeptid mit der GenBank-Zugangsnummer AKM18207 oder ein funktionelles Fragment davon mit der gewünschten GtfC-Typ 4,6-α-Glucanotransferase-Aktivität ist.

8. Verfahren oder Teig nach einem der vorhergehenden Ansprüche, wobei das GtfC-Typ Enzym ein Polypeptid ist, umfassend eine Aminosäuresequenz mit mindestens 80 % Identität zu Sequenz 1 (Fig. 1), vorzugsweise umfassend eine Aminosäuresequenz mit mindestens 80 % Identität zu Sequenz 2 (Fig. 2).

9. Verfahren oder Teig nach Anspruch 8, wobei das Polypeptid die konservierten Motive I, II, III und IV der GTFC-ähnlichen Mitglieder der GH70-Familie umfasst, wobei die Motive die folgenden Aminosäuresequenzen haben:
Motiv I: [M/E/L]DLVPNQ, vorzugsweise LDLVPNQ
Motiv II: GFRIDAA[S/G/T]H[Y/F]D, vorzugsweise GFRIDAATHFD
Motiv III: HLSYIESY[K/S/Q/T][S/N][E/V/A/K], vorzugsweise HLSYIESYTSK
Motiv IV: FV[N/T]NHDQEKNR[V/I]N[Q/N/T], vorzugsweise FVNNHDQEKNRVNT.

10. Verfahren oder Teig nach einem der vorhergehenden Ansprüche, wobei der Teig durch Kombinieren von Mehl, Wasser, Hefe, GtfC-Typ 4,6-α-Glucanotransferase-Enzym und optional anderen Backzutaten hergestellt wird.

11. Verfahren oder Teig nach einem der vorhergehenden Ansprüche, wobei der Teig ein Weizenmehlteig ist.

12. Verfahren oder Teig nach einem der Ansprüche 1-11, wobei der Teig ein glutenfreier oder glutenreduzierter Teig ist, wobei der Teig vorzugsweise natives Kartoffelprotein in einer Menge von 0,2 bis 8 Gew.-%, bezogen auf den gesamten Teig, umfasst.

13. Ein gebackenes Lebensmittelprodukt, erhältlich durch ein Verfahren nach einem der Ansprüche 1, 3-12 oder durch Backen eines Teigs nach einem der Ansprüche 2-12.

14. Ein gebackenes Lebensmittelprodukt, erhalten durch Backen eines mehlhaltigen Teigs, umfassend ein lineares α-Glucan, umfassend alternierende α(1→6)/α(1→4) glycosidische Bindungen und keine α(1→ 4,6)-Verzweigungspunkte, weniger als 0,5 % aufeinanderfolgende α(1→6) glycosidische Bindungen und weniger als 0,5 % aufeinanderfolgende α(1→ 4) glycosidische Bindungen.

15. Verfahren zur Verlängerung der Haltbarkeit eines gebackenen Lebensmittelprodukts, vorzugsweise eines Brots, umfassend Einbringen eines GtfC-Typ 4,6-α-Glucanotransferase-Enzyms in einen mehlhaltigen Brotteig, das in der Lage ist, eine Maltoseeinheit von einem Polysaccharid- oder Oligosaccharidsubstrat auf das nichtreduzierende Ende eines Oligosaccharidakzeptors über eine neue α(1→6)-Bindung zu übertragen, ohne α(1→4,6)-Verzweigungspunkte zu bilden, und wobei das GtfC-Typ Enzym ein Temperaturoptimum im Bereich von 50-70 °C hat, bestimmt bei pH 6 mit Amylose V als Substrat, und Backen des Teigs.

16. Verwendung eines GtfC-Typ 4,6-α-Glucanotransferase-Enzyms als Altbackenwerden verhinderndes Mittel in einem gebackenen Teigprodukt, wobei das GtfC-Typ 4,6-α-Glucanotransferase-Enzym in der Lage ist, eine Maltoseeinheit von einem Polysaccharid- oder Oligosaccharidsubstrat auf das nichtreduzierende Ende eines Oligosaccharidakzeptors über eine neue α(1→6)-Bindung zu übertragen, ohne α(1→4,6)-Verzweigungspunkte zu bilden, und wobei das GtfC-Typ Enzym ein Temperaturoptimum im Bereich von 50-70 °C hat, bestimmt bei pH 6 mit Amylose V als Substrat.

17. Verwendung eines GtfC-Typ 4,6-α-Glucanotransferase-Enzyms, um mindestens eine Eigenschaft eines gebackenen Teigprodukts zu verbessern oder zu erhalten, vorzugsweise um eines oder mehrere von Laibvolumen, Krumenstruktur, Krumenelastizität, Krumenfederung, Teigstärke, Teigstabilität und Krumenweichheit zu verbessern oder zu erhalten, wobei das GtfC-Typ 4,6-α-Glucanotransferase-Enzym in der Lage ist, eine Maltoseeinheit von dem Polysaccharid- oder Oligosaccharidsubstrat auf das nichtreduzierende Ende eines Oligosaccharidakzeptors über eine neue α(1→6)-Bindung zu übertragen, ohne α(1→4,6)-Verzweigungspunkte zu bilden, und wobei das GtfC-Typ Enzym ein Temperaturoptimum im Bereich von 50-70 °C hat, bestimmt bei pH 6 mit Amylose V als Substrat.

## Revendications

1. Procédé de préparation d'un produit alimentaire cuit au four par cuisson d'une pâte farineuse, comprenant l'incorporation dans la pâte d'une enzyme 4,6-α-glucanotransférase de type GtfC, dans lequel l'enzyme de type GtfC (i) est capable de transférer un fragment maltose d'un substrat polysaccharidique ou oligosaccharidique à l'extrémité non réductrice d'un accepteur oligosaccharidique via une nouvelle liaison α(1→6) sans formation de points de ramification α(1→4,6), et (ii) a un optimum d'activité, déterminé à pH 6 avec de l'amylose V en tant que substrat, dans la plage de températures allant de 50 à 70 °C.

2. Pâte farineuse comprenant une enzyme 4,6-α-glucanotransférase de type GtfC, dans laquelle l'enzyme de type GtfC (i) est capable de transférer un fragment maltose d'un substrat polysaccharidique ou oligosaccharidique à l'extrémité non réductrice d'un accepteur oligosaccharidique via une nouvelle liaison α(1→6) sans formation de points de ramification α(1→4,6), et (ii) a un optimum d'activité, déterminé à pH 6 avec de l'amylose V en tant que substrat, dans la plage de températures allant de 50 à 70 °C.

3. Procédé ou pâte selon la revendication 1 ou 2, dans lequel l'enzyme de type GtfC a un optimum d'activité dans la plage de températures allant de 55 à 65 °C.

4. Procédé ou pâte selon l'une quelconque des revendications précédentes, dans lequel l'enzyme de type GtfC a une activité de 4,6-α-glucanotransférase résiduelle d'au moins 80 % après incubation de l'enzyme dans un tampon citrate de sodium 25 mM contenant du CaCl₂ 1 mM à une température de 60 °C et à pH 6 pendant 60 minutes.

5. Procédé ou pâte selon l'une quelconque des revendications précédentes, dans lequel l'enzyme de type GtfC a un optimum d'activité dans la plage de pH allant de 4 à 7,5, de préférence de 5,0 à 7,0, mieux encore de 5,5 à 6,5, dans un tampon citrate de sodium 25 mM contenant du CaCl₂ 1 mM à une température de 40 °C avec de l'amylose V en tant que substrat.

6. Procédé ou pâte selon l'une quelconque des revendications précédentes, dans lequel l'enzyme de type GtfC est incorporée dans la pâte en une quantité de 100 à 20 000 U par kg du poids total d'amidon dans la pâte, de préférence en une quantité de 500 à 15 000 U/kg d'amidon, dans lequel l'activité, exprimée en Unités, est définie comme étant la quantité d'amylose V (mg) convertie par mg d'enzyme par minute, déterminée dans un tampon citrate de sodium 25 mM contenant du CaCl₂ 1 mM à une température de 60 °C et à pH 6 avec de l'amylose V en tant que substrat.

7. Procédé ou pâte selon l'une quelconque des revendications précédentes, dans lequel l'enzyme de type GtfC est un polypeptide ayant le numéro d'accès GenBank AKM18207, ou un fragment fonctionnel de celui-ci ayant l'activité de 4,6-α-glucanotransférase de type GtfC souhaitée.

8. Procédé ou pâte selon l'une quelconque des revendications précédentes, dans lequel l'enzyme de type GtfC est un polypeptide comprenant une séquence d'acides aminés ayant une identité d'au moins 80 % avec la Séquence 1 (Figure 1), de préférence comprenant une séquence d'acides aminés ayant une identité d'au moins 80 % avec la Séquence 2 (Figure 2).

9. Procédé ou pâte selon la revendication 8, dans lequel le polypeptide comprend les motifs conservés I, II, III et IV des membres de type GTFC de la famille GH70, dans lequel les motifs ont les séquences d'acides aminés suivantes :
Motif I : [M/E/L]DLVPNQ, de préférence LDLVPNQ
Motif II : GFRIDAA[S/G/T]H[Y/F]D, de préférence GFRIDAATHFD
Motif III : HLSYIESY[K/S/Q/T][S/N][E/V/A/K], de préférence HLSYIESYTSK
Motif IV : FV[N/T]NHDQEKNR[V/I]N[Q/N/T], de préférence FVNNHDQEKNRVNT.

10. Procédé ou pâte selon l'une quelconque des revendications précédentes, dans lequel la pâte est préparée par combinaison de farine, d'eau, de levure, d'enzyme 4,6-α-glucanotransférase de type GtfC, et éventuellement d'autres ingrédients de boulangerie.

11. Procédé ou pâte selon l'une quelconque des revendications précédentes, dans lequel la pâte est une pâte à la farine de blé.

12. Procédé ou pâte selon l'une quelconque des revendications 1 à 11, dans lequel la pâte est une pâte sans gluten ou à teneur réduite en gluten, de préférence dans lequel la pâte comprend de la protéine de pomme de terre native en une quantité de 0,2 à 8 % p/p de la pâte totale.

13. Produit alimentaire cuit au four pouvant être obtenu par un procédé selon l'une quelconque des revendications 1 et 3 à 12 ou par cuisson d'une pâte selon l'une quelconque des revendications 2 à 12.

14. Produit alimentaire cuit au four obtenu par cuisson d'une pâte farineuse comprenant un α-glucane linéaire comprenant des liaisons glycosidiques α(1→6)/α(1→4) alternées et n'ayant pas de points de ramification α(1→4,6), moins de 0,5 % de liaisons glycosidiques α(1→6) consécutives et moins de 0,5 % de liaisons glycosidiques α(1→4) consécutives.

15. Procédé pour augmenter la durée de conservation d'un produit alimentaire cuit au four, de préférence d'un pain, comprenant l'incorporation dans une pâte à pain farineuse d'une enzyme 4,6-α-glucanotransférase de type GtfC qui est capable de transférer un fragment maltose d'un substrat polysaccharidique ou oligosaccharidique à l'extrémité non réductrice d'un accepteur oligosaccharidique via une nouvelle liaison α(1→6) sans formation de points de ramification α(1→4,6), et dans lequel l'enzyme de type GtfC a une température optimale, déterminée à pH 6 avec de l'amylose V en tant que substrat, dans la plage de températures allant de 50 à 70 °C, et la cuisson de la pâte.

16. Utilisation d'une enzyme 4,6-α-glucanotransférase de type GtfC en tant qu'agent ralentisseur de rassissement dans un produit à base de pâte cuit au four, dans laquelle ladite enzyme 4,6-α-glucanotransférase de type GtfC est capable de transférer un fragment maltose d'un substrat polysaccharidique ou oligosaccharidique à l'extrémité non réductrice d'un accepteur oligosaccharidique via une nouvelle liaison α(1→6) sans formation de points de ramification α(1→4,6), et dans laquelle l'enzyme de type GtfC a une température optimale, déterminée à pH 6 avec de l'amylose V en tant que substrat, dans la plage de températures allant de 50 à 70 °C.

17. Utilisation d'une enzyme 4,6-α-glucanotransférase de type GtfC pour améliorer ou préserver au moins une propriété d'un produit à base de pâte cuit au four, de préférence pour améliorer ou préserver un ou plusieurs parmi le volume du pain, la structure de la mie, la résilience de la mie, l'élasticité de la mie, la force de la pâte, la stabilité de la pâte, le moelleux de la mie, dans laquelle ladite enzyme 4,6-α-glucanotransférase de type GtfC est capable de transférer un fragment maltose d'un substrat polysaccharidique ou oligosaccharidique à l'extrémité non réductrice d'un accepteur oligosaccharidique via une nouvelle liaison α(1→6) sans formation de points de ramification α(1→4,6), et dans laquelle l'enzyme de type GtfC a une température optimale, déterminée à pH 6 avec de l'amylose V en tant que substrat, dans la plage de températures allant de 50 à 70 °C.
